# EUROPEAN PATENT SPECIFICATION

(11) **EP 3 498 261 B1**
(45) Date of publication and mention of the grant of the patent: **07.01.2026**
(21) Application number: 18153032.0
(22) Date of filing: 23.01.2018
(51) Int. Cl.: A61K 31/255, A61K 31/315, A61K 9/00, A01N 1/00, A61K 8/27, A61K 8/46, A61Q 17/00, A61K 47/20, A61L 26/00, A61P 31/04, A61P 17/02, A01N 41/02

(54) **COMPOSITION COMPRISING ZINC AND SURFACTANTS FOR USE IN REMOVING BACTERIAL BIOFILMS AND TREATING TISSUES**
ZUSAMMENSETZUNG ENTHALTEND ZINKGLUCONAT UND OBERFLÄCHENAKTIVE SUBSTANZEN ZUR VERWENDUNG BEI DER ENTFERNUNG BAKTERIELLER BIOFILME UND ZUR BEHANDLUNG VON GEWEBEN
COMPOSITION COMPRENANT DU GLUCONATE DE ZINC ET DES TENSIOACTIFS POUR SON SON UTILISATION DANS L'ÉLIMINIATION DES BIOFILMS BACTÉRIENS ET LE TRAITEMENT DE TISSUS

(30) Priority: 14.12.2017 US 201762598575 P
(43) Date of publication of application: 19.06.2019
(73) Proprietor: Infection Elimination Services LLC, Pelzer, SC 29669 (US)
(72) Inventor: Palmer, Charles F., Greenville, SC 29604-8915 (US); Campbell, Thomas William, Greenville, SC 29604-8915 (US)
(74) Representative: Reddie & Grose LLP

(56) References cited:
- WO-A1-2014/134731
- WO-A2-2004/010958
- US-A1- 2018 055 808
- US-B1- 6 723 688
- DATABASE GNPD [online] MINTEL; May 2013 (2013-05-01), ANONYMOUS: "Purifying Cleansing gel", XP002782413, retrieved from www.gnpd.com Database accession no. 2060172
- DATABASE GNPD [online] MINTEL; January 2016 (2016-01-01), ANONYMOUS: "Purifying Cleansing Bar", XP002782414, retrieved from www,gnpd.com Database accession no. 3681203

## Description

### BACKGROUND

This invention is related to a treatment mixture for use in improving the healing rate of infected wounds or sores on humans or animals, that are otherwise slow to heal, by usual treatments. More specifically, the present invention is related to a mixture of surfactants and zinc salts and their use in removing biofilms, especially those from body tissue or materials in contact with body tissue such as implants.

The historical view of bacteria is that they are free-living organisms easily kept in check by antibiotics, however, scientists are now realizing that bacteria spend most of their lives in colonies, or biofilms, even in the human body. Biofilms are communities of bacteria in self-produced slime and may be found almost anywhere that solids and liquids meet, whether in nature, in hospitals or in industrial settings. According to the United States' Centers for Disease Control, biofilms are implicated in more than 80% of chronic inflammatory and infectious diseases caused by bacteria, including ear infections, gastrointestinal ulcers, urinary tract infections and pulmonary infections in cystic fibrosis patients. It is widely thought that in their natural habitat most bacteria live as a community and attach to surfaces as biofilms and that many infections in humans are related to biofilms. While single bacteria may be treatable with antibiotics, the films can be 1,000 times more resistant and most can only be removed surgically.

Bacteria that form biofilms occasionally infect implants such as pacemakers, stents, and artificial joints. These biofilm sites periodically shed bacteria, often referred to in the art as adventurers, which can ignite acute infections and fever. While antibiotics can knock out these free-swimming bacteria and temporally calm down the infection, the biofilm remains untouched. The only permanent solution is removal of the biofilm-coated device and replacement with a new sterilized implant.

A permanent bacterial biofilm in the sinuses can ignite an immune response leading to chronic sinus infections, with symptoms including fever and cold-like symptoms. So far, the most effective treatment is to surgically remove the affected tissue.

Bacteria also form permanent, mostly lifelong, biofilms in the mucus-filled lungs of cystic fibrosis patients and are responsible for the chronic lung infections that lead to early death. Although long-lasting antibiotic treatment helps, it cannot eradicate the infection completely.

Biofilms are difficult to eradicate with conventional antimicrobial treatments since they are far more resistant to antibiotics than planktonic, or free-floating adventurer cells. Biofilms also pose a persistent problem in many industrial processes, including drinking water distribution networks and manufacturing environments.

The problem with a chronic infection is that the immune system attempts to clear the infection but is unable to. The longer the chronic infection goes on, the more damage there will be to tissue at the site of the infection because the immune response often involves the release of toxic compounds that have no effect on biofilms but can damage the surrounding tissues.

In one reported observation, over a period of about six hours, a single bacterium laid down a glue to attach itself to a surface, then divided into daughter cells, making certain to cement each daughter to itself before splitting in two. The daughters continued to divide until they formed a cluster, like a brick and mortar building, at which point the bacteria secreted a protein encasing the cluster like the shell of a building. The clusters are separated by micro-channels that may allow nutrients in and waste out.

Bed sores, also known as pressure ulcers, pressure sores, or decubitus ulcers are skin lesions which can be caused by friction, humidity, temperature, incontinence, medication, shearing forces, age and unrelieved pressure. Any part of the body may be affected, however, bony or cartilaginous areas, such as the elbows, knees, ankles and sacrum are most commonly affected. If discovered early, bedsores are treatable. However, they may sometimes be fatal. According to health authorities in the UK and USA, bedsores are the second iatrogenic cause of death, after adverse drug reactions causing hospitals to spend about $5 billion annually for treatment of pressure ulcers.

Biofilms are one of the most common reasons for delayed healing in pressure ulcers. Biofilm formation occurs rapidly in wounds and stalls healing by keeping the wound inflamed. Frequent debridement and antimicrobial dressings are needed to control the biofilm. Infection prevents healing of pressure ulcers. Symptoms of infection in a pressure ulcer include slow or stalled healing and pale granulation tissue. Infection can expand from local to systemic. Symptoms of systemic infection include fever, pain, redness, swelling, warmth of the area, and purulent discharge. Additionally, infected wounds may have a gangrenous smell, be discolored, and may eventually exude even more pus. In order to eliminate this problem, it is imperative to apply antiseptics at once. Hydrogen peroxide, a near-universal toxin, is not recommended for this task as it increases inflammation and impedes healing. Systemic antibiotics are not recommended in treating local infection in a pressure ulcer, as it can lead to bacterial resistance. They are only recommended if there is evidence of advancing cellulitis, osteomyelitis, or bacteremia.

Surfactants with detergency are known to remove a number of water insoluble materials from hard surfaces such as oily materials, grassy materials, proteinaceous materials and dirt based materials. Surfactants are usually organic compounds that are amphiphilic, meaning they contain both hydrophobic moieties, often referred to as their tails, and hydrophilic moieties, often referred to as their heads. Surfactants will diffuse into water and adsorb at interfaces between air and water or at the interface between oil and water, in the case where water is mixed with oil. The water-insoluble hydrophobic group may extend out of the bulk water phase, into the air or into the oil phase, while the water-soluble head group remains in the water phase.

Detergents have also been used to decellularise organs with limited success. This process maintains a matrix of proteins that preserves the structure of the organ and often the microvascular network. The process has been successfully used to prepare organs such as the liver and heart for transplant in rats. Pulmonary surfactants are also naturally secreted by type II cells of the lung alveoli in mammals.

Other approaches toward treating biofilms are known. U.S. Pat. No. 8,753,662 teaches methods of inhibiting biofilm formation or reducing biofilms in a subject or on a device or surface by administering a charged compound such as a polyamino acid to a subject, device or surface. The invention also relates to compositions for inhibiting biofilm formation or reducing biofilms. U.S. Pat. No. 8,748,617 discloses the use of amide compounds or salts thereof and biofilm inhibitor, biofilm remover, and disinfectant containing the same. The disclosure provides an amide compound and salt thereof that is capable of inhibiting biofilm formation or removing deposited biofilms. U.S. Pat. No. 8,747,872 relates to methods and compositions for treating pulmonary infection. In particular, it provides nanoemulsion compositions and methods of using the same to treat bacteria associated with biofilms such as those found in pulmonary infections. Compositions and methods of the invention find use in, among other things, clinical settings for use as therapeutic and preventative medicine, industrial applications, and research applications.

The prior art cited above shows materials designed to kill bacteria or inhibit biofilm formation. It also shows that certain detergent surfactants are known to lyse cell membranes and tissues by disorganizing the membrane's lipidic bilayer, which would damage healthy tissue, however, they are marginally effective. The milder detergents disclosed in the art such as octyl thioglucoside, octyl glucoside or dodecyl maltoside that are used to solubilize membrane proteins such as enzymes and receptors without denaturing them are expensive and not widely available.

It is clear that there is a need for an effective treatment mixture for use in removing bacterial biofilms, especially those in bedsores or on implants, to reduce the mortality rate due to infections, both internal and external to the body. Further, a treatment mixture that avoids systemic antibiotics would have advantages of lower treatment cost, the avoidance of adverse reactions to the medications, and avoid the development of bacterial resistance to antibiotics. A nonsurgical treatment to remove biofilms would likely have lower treatment costs, reduced risk of complications, and reduced need to remove healthy tissue along with the infected tissue.

In spite of the ongoing effort there is still a desire for a treatment mixture for use in disrupting biofilms thereby releasing the bacteria therefrom to allow natural mitigation of infection or increased access to systemic or localized antibiotics. There is also an ongoing desire for improved tissue healing in concert with disruption of biofilms. Such an improvement is provided herein.
US 6723688 B1 discloses compositions for cleansing "that are not irritating to human skin. A composition is disclosed that includes at least 2% of a mixture of a salt of an alkyl sulfoacetate and a salt of an ethoxylated alkyl sulfosuccinate, at least 3% of a salt of an ethoxylated alkyl sulfate, at least 1% of an amphoteric surfactant, at least 0.05% of an alkyl glucoside, and at least 0.005% of a phospholipid. A composition for cleansing is disclosed that includes about 2% to 70% of a mixture of a salt of an alkyl sulfoacetate and a salt of an ethoxylated alkyl sulfosuccinate, about 3% to 40% of a salt of an ethoxylated alkyl sulfate, about 1% to 40% of an amphoteric surfactant, about 0.05% to 10% of an alkyl glucoside, and about 0.005% to 10% of a phospholipid. A cleansing composition is further disclosed that includes about 41% of a mixture of alkyl sulfoacetate and ethoxylated alkyl sulfosuccinate, about 34% of an ethoxylated alkyl sulfate, about 22% of an amphoteric surfactant, about 2% of a alkyl glucoside, and about 0.5% of a phospholipid. A composition for cleansing is also described which includes about 2% to 50% of a mixture of a salt of a sodium lauryl sulfoacetate and Disodium Laureth Sulfosuccinate, about 3% to 35% of Ammonium Laureth Sulfate, about 1% to 30% of Cocamidopropyl Betaine, about 0.05% to 8% of Coco-Glucoside and Coconut Oil, and about 0.005% to 5% of a phospholipid. A method of cleaning is also described, which includes contacting a surface to be cleansed with the composition of the invention. The composition can be used in a variety of personal care or household cleansers, such as contact lens care products, shampoos, soaps, body washes, mouthwashes, toothpastes, oral rinses, facial and wound cleansers, eye makeup removers, laundry and dishwashing detergents, and others."
WO 2014/134731 A1 discloses "[c]ompositions comprising chelating agents, metal ion salts, gelling agents or a buffer, antimicrobials, antibiofilm agents and a pH adjuster or a buffer for the prevention and treatment of wound infections and food-borne diseases involving bacterial biofilms are disclosed. The anti-infective properties of a composition include reduction or killing of anaerobic/aerobic/facultative gram-negative and gram-positive wound infection associated bacteria occurring in polymicrobial biofilms. The composition may be in the form of lotion, cream, ointment, dressing, bandage, rinse, soak, gel, spray, or other suitable forms, including certain devices. Additionally, the invention offers an efficient method of delivering the formulated composition containing one or two chelating agents or chelating agents alone or in combination with a metal ion salt using either a nanoparticle or other efficient delivery systems."

### SUMMARY OF THE INVENTION

It is an object of the invention to provide a treatment mixture for use in disrupting biofilms and to provide improved tissue healing.

It is another object of the invention to provide a treatment mixture for use in disrupting biofilms without adverse effect on surrounding healthy tissue and with advantegous effects on tissue healing.

A particular feature of the present invention is the simplicity in use which does not require significant medical training and can be done by a patient or untrained care provider.

These and other embodiments, as will be realized, are provided in a treatment mixture for use in the removal of bacterial biofilms on a human or animal body, wherein said treatment mixture can be applied and wherein said biofilm is integral to a bedsore on a human or animal body wherein the treatment mixture comprises: sodium laureth sulfate, 26% solution in water, at an amount of 34 wt% of the solids; sodium lauryl sulfoacetate and disodium laureth sulfosuccinate, 25% solution in water, at
an amount of 33 wt% of the solids;
zinc gluconate powder, at an amount of 14 wt% of the solids;
sorbitol, 70% solution, at an amount of 3 wt% of the solids;
cocodimethylaminopropylbetaine, 35% in water, at an amount of 11 wt% of the solids; imidazolidinyl urea, at an amount of 1 wt% of the solids;
tetrasodium EDTA dihydrate solution to adjust pH to 7.4, at an amount of 4 wt% of the
   solids; and
water.

Yet another embodiment is provided in a treatment mixture for use in the removal of bacterial biofilms comprising:
applying a treatment mixture to said biofilm wherein said treatment mixture comprises:
sodium laureth sulfate, 26% solution in water, at an amount of 34 wt% of the solids;
sodium lauryl sulfoacetate and disodium laureth sulfosuccinate, 25% solution in water, at
   an amount of 33 wt% of the solids;
zinc gluconate powder, at an amount of 14 wt% of the solids;
sorbitol, 70% solution, at an amount of 3 wt% of the solids;
cocodimethylaminopropylbetaine, 35% in water, at an amount of 11 wt% of the solids; imidazolidinyl urea, at an amount of 1 wt% of the solids;
tetrasodium EDTA dihydrate solution to adjust pH to 7.4, at an amount of 4 wt% of the
   solids; and
water;
treating said biofilm to at least one procedure selected from the group consisting of rinsing with water and allowing said biofilm to dry; and
re-applying a treatment mixture to said biofilm.

### DESCRIPTION

The instant invention is directed to an improved treatment mixture for use in removing bacterial biofilms which is particularly suitable for treating infected wounds or implants. More specifically, the present invention is directed to a mixture of surfactants which is effective in the disruption of the biofilm matrix thereby allowing for the release of bacteria for subsequent treatment and the mixture includes a zinc salt to aid in tissue healing.

While not limited to any theory, the treatment mixture of the present invention provides a mixture of surfactants which is believed to disrupt and disperse the biofilm and zinc salt which facilitates tissue healing thereby allowing the body's natural defenses to attack the released bacteria and facilitate healing of the underlying tissue. Alternatively, the released bacteria are more readily treatable with antibiotics either systemically or locally applied.

Disclosed herein is a treatment mixture comprising active components with an ethoxylated component defined by Formula I and at least one of Formula II or Formula III and preferably both Formula II and Formula III and zinc cation as a salt. Formula I is a salt of alkyleth sulfate defined by:

R¹-(OCHR²CH₂)ₙ-OSO₃X¹ Formula I

wherein:
R¹ is a branched or unbranched alkyl of 8-24 carbons, more preferably 10-18 carbons and most preferably 12-14 carbons;
R² is hydrogen or methyl;
n is an integer of 2 to 30, preferably 4-10;
each X¹ is independently a counter ion preferably selected from alkali metal or ammonium and preferably sodium or potassium;
Formula II is a salt of an alkyl sulfoacetate defined by:

   R⁴OC(O)CH₂SO₃X² Formula II
wherein R⁴ is a branched or unbranched alkyl of 8-24 carbons, more preferably 10-18 carbons and most preferably 12-14 carbons;
and X² is a counter ion preferably selected from alkali metal and preferably sodium or potassium;
Formula III is a salt of an alkyl sulfosuccinate defined by: 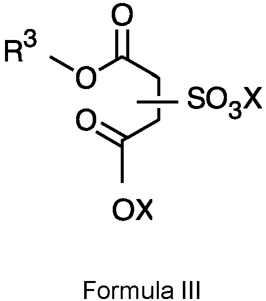
wherein:
   R³ is the branched or unbranched alkyl group of an alkyl alcohol with the alcohol hydrogen replaced with the sulfosuccinate wherein the alkyl has 8-24 carbons, more preferably 10-18 carbons and most preferably 12-14 carbons, and
   each X is independently a counter ion preferably selected from alkali metal metal or ammonium and preferably sodium or potassium.

Though not limited to any theory, the ethoxy groups of Formula I are believed to facilitate rupture of the cell membranes of the biofilm thereby allowing the other components, represented by Formula II and Formula III, to more effectively solubilize and disperse the fragmented biofilm. A particularly preferred compound of Formula I is sodium laureth sulfate.

A particularly preferred compound of Formula II is sodium lauryl sulfoacetate.

A particularly preferred compound of Formula III is disodium lauryl sulfosuccinate.

The zinc is added as a salt wherein the zinc is present as Zn²⁺. Particularly preferred zinc salts include zinc gluconate, zinc sulfate, zinc chloride or zinc acetate. The effective amount of zinc is 0.1 to 0.5 wt% of the solids. Below about 0.1 wt% the effectiveness diminishes and above about 0.5 wt% the effect is not sufficiently increased to justify the additional ingredient. Zinc salts in a surfactant system are expected to be rendered ineffective due to the ligation of the zinc cation by the surfactant. This is especially so when the composition is dried down. Contrary to expectations the combination of zinc cation and surfactant composition has a synergistic effect wherein the surfactant disrupts the biofilm and maintains the zinc in effective concentration to effect tissue revitalization.

The treatment mixture is preferably an aqueous solution and may comprise additional adjuvants such as humectants or hygroscopic materials; rheology aids such as foam enhancers or foam stabilizers; acids or bases to adjust pH; chelates or chelating agents; preservatives to inhibit microbial growth such as antimicrobial medicines or antibiotic; viscosity control agents or additional surfactant.

While not limited to theory, humectants are believed to assist in wetting the biofilm thereby improving the ability of the active components to disrupt and disperse the biofilm. Hygroscopic materials can impart a moisturizing effect to the treated tissues and by attracting water may improve the removal of the biofilms by subsequent rinsings. Polyols and polyethoxylated polyols are particularly preferred humectants. Particularly preferred polyols include sorbitol, glycerine, and other simple sugars. Particularly preferred polyethoxylated polyols include glycereth-26 (polyethylene 26 glycerin), sorbitol ethoxylate, and polyethylene glycol.

Rheology aids assist in controlling the viscosity, or rheology, of the treatment mixture thereby optimizing residence time of the treatment mixture at the site of the biofilm. In some applications the treatment mixture may have a low viscosity suitable for application as a flowing liquid or spray. In other applications the treatment mixture may have a higher viscosity for application as a gel or cream which remains at the site with minimal flowing until rinsed. In other applications the treatment mixture may foam thereby insuring a longer residence time with optional effervescence to provide refreshment of active components. Foam stabilizers are known to stabilize foams and the foam strength may be related to biofilm removal efficacy. Sorbitol, glycerin, and ethoxylated glycerin or ethoxylated sorbitol are some suitable hygroscopic and foam stabilizing additives. Thickeners or other viscosity control agents may be added to gel the surfactant mixture to hold it in place on the sore. The treatment mixture may be rheological having shear thinning properties thereby allowing for spray applications, such as through a nozzle, with higher viscosity after application for increased residence time. Particularly preferred rheology aids include foam enhancers such as cocoamidopropyl betaine, cocoamide, cocoamide MEA, and cocoamide DEA.

The pH of the treatment mixture can be adjusted by acids and bases wherein the acid or base may have additional functionality such as functioning as a chelating agent. Particularly preferred acids and bases are those that are biologically inert. Lactic, citric, and ascorbic acids are a particularly suitable acids for lowering pH and tetrasodium ethylenediaminetetraacetic acid (EDTA) and sodium citrate are particularly suitable bases for raising pH.

The treatment mixture may include stabilizers and preservatives, particularly, if microbial growth of the mixture is a concern or for storage or transport. Antimicrobial medicines or antibiotics to kill the bacteria or other microbes present may speed wound healing. Particularly preferred stabilizers or preservatives include imidazolidinyl urea, parabens, methylchloroisothiazolinone, and methylisothiazolinone. Topical antibiotics are most preferred. Particularly suitable antibiotics include bacitracin zinc, neomycin sulfate, and polymyxin B sulfate.

The concentration of active components can be relatively low and still be effective. A concentration effective amount is about 0.025 wt % to about 30 wt % solids in water with solids being the total sum of the active components. Adjuvants are preferably about 0.025 wt% to about 20 wt%. Below 0.025 wt% active components the effectiveness of the mixture is insufficient to disrupt the biofilm at a reasonable rate thereby requiring excess flow of material. Above about 30 wt% active components the room temperature viscosity is too high to easily handle the product and the volume of water is insufficient to solubilize and remove the disrupted material. The concentration of active components in the treatment solution should be chosen so that they are effective at removing the biofilm in a small number of rinsings, but not so concentrated as to cause excessive irritation or damage to the nearby human tissue. A concentration of at least about 5 wt% active component to no more than about 15 wt% is preferred with about 10 wt % of active components being optimal for most applications. The concentration for subsequent rinsings may be adjusted up or down as desired. A higher concentration of surfactant will increase the detergency and rate of biofilm removal or destruction, but will increase the chance of healthy tissue irritation or damage.

The treatment mixture is suitable for use in a method for removal of bacterial biofilms from living tissue and hard surfaces by physical removal through the action of a mild surfactant solution in a water-based cleansing solution. This biofilm removal method is effective yet inexpensive, based on readily available surfactants, leaves peripheral patient tissue largely unaffected by the treatment, and is very gentle and well tolerated by the patient.

The present invention is a treatment mixture for use in increasing the rate of healing of sores on humans or animals, especially pressure sores, comprising the application of an effective amount of an aqueous solution of surfactants for a length of time suitable to effect a cure. Not wishing to be bound by conjecture, it is believed that the efficacy of the inventive surfactant mixture is largely due to its ability to destroy and/or remove the biofilm from the sore and allow the body's healing mechanisms to function unimpeded by the biofilm and the bacterial toxins.

It is expected that different sores will have different biofilms, and thus the amount of treatment surfactant mixture, its concentration, and length of treatment time to destroy the biofilm and promote healing will vary. In addition, other factors related to wound healing rates such as blood supply, depth of the sore, health of the individual, etc., will affect the rate of healing.

The biofilm removal may take more than one application of surfactant solution, different methods of surfactant application, and may take several days of treatments. The surfactants are chosen to be effective in removing the biofilm as well as to minimize adverse effects to the living non-bacterial tissue adjoining the biofilm.

Suitable surfactants for use in the treatment mixture and combinations thereof preferably exhibit both low toxicity and irritation. They must also not destroy the human tissue near the biofilm to be removed. Some anionic surfactants that function well to assist in the removal of biofilms include alcohol ethoxylate sulfates, alcohol sulfoacetates, and alcohol ethoxylate sulfosuccinates that are used in personal care applications. Anionic alcohol sulfates, alcohol ethoxylate carboxylates, or sulfonates may also be effective. These anionic surfactants are chosen to assist in effective biofilm removal or destruction and cause little irritation or damage to healthy tissue. In a preferred embodiment a small amount of treatment mixture is applied to a portion of tissue which is not infected to insure no reaction will occur.

Nonionic or amphoteric surfactants may be combined with the treatment mixture to improve the detergency, though they can also be irritating.

The inventive mixture of surfactants and additives may be placed in contact with the sore to be healed via a number of methods. A suitable method of applying the surfactant mixture to the sore to be treated is to soak the aqueous surfactant mixture into a cotton gauze pad and placing the wet gauze pad onto the sore. The gauze pad may be replaced as needed to follow medical protocols for wound dressing and healing. The gauze pad may be allowed to dry before removal as in a wet to dry pack wound treatment.

Other suitable methods of applying the surfactant mixture to the sore include spraying the aqueous surfactant mixture directly onto the sore to be treated or applying a thickened solution of the surfactants to the sore.

In another method, the surfactant mixture is added to water to give the desired concentration, placed in a water-stream-generating container such as a squirt bottle, and the water solution applied to the biofilm with mild pressure so that the surfactant solution flows into the wound over several seconds. A suitable flow rate is approximately one ounce of surfactant solution per second. A greater flow rate is acceptable, but should be adjusted so that the majority of the surfactant solution contacts the biofilm so that it may be physically removed by the action of the surfactants. The minimum flow rate is not defined, but since generally higher flow rates of surfactant solution result in greater biofilm removal rates, the flow rate should be maximized to reduce the time required and number of applications for complete biofilm removal. The surfactant solution application may be repeated as often as tolerated by the patient or as practical, up to several times per day.

There is no defined maximum or minimum amount of surfactant solution to be applied to a particular biofilm. In general, the larger the biofilm, the more treatment solution will be necessary. Longer application of more surfactant solution will remove more biofilm, and thus reduce the total treatment time. Removal of a biofilm internal to the body might preferably be completed by a single extended treatment to minimize exposure of internal organs to further infection. It is preferable to rinse the biofilm with water after application of the treatment mixture and allowing the biofilm to dry before reapplication.

The temperature of the treatment mixture is not critical, but if it is to be used on living tissue, the temperature should be adjusted close to body temperature to minimize any discomfort or damage.

The biofilms may be either internal or external to the body, and may be either on humans or animals or plants or hard surfaces. If the biofilm is on a hard surface the application rate and pressure may be increased to increase the biofilm removal rate.

### EXAMPLES

### Treatment Mixture A

The following ingredients were combined in the amounts shown. Care was taken to avoid air entrainment and foaming during the mixing operation.

| Ingredient | Amount (grams) | Concentration (gms of ingredient per gm of solution ) delivered by supplier | solids (active ingredient gms) | Fraction of total solids in product |
|---|---|---|---|---|
| (Glycereth-26 CAS 31694-55-0 | 183.13 | 1 | 183.13 | 0.16 |
| Sorbitol 70% solution | 52.32 | 0.7 | 36.63 | 0.03 |
| Sodium Laureth Sulfate CAS 68585-34-2 (26% solution in water) | 1465 | 0.26 | 380.90 | 0.33 |
| Sodium Lauryl Sulfoacetate (CAS 1847-58-1) and Disodium Laureth Sulfosuccinate (CAS 39354-45-5) = STEPAN MILD^{©} LSB (25% solution in water) | 1465 | 0.25 | 366.25 | 0.32 |
| Cocodimethylaminopropylbetaine, CAS 70851-07-9, 35% in water | 366.25 | 0.35 | 128.19 | 0.11 |
| Imidazolidinyl urea (preservative) (Germal 115, CAS 39236-46-9) | 14.60 | 1 | 14.60 | 0.01 |
| USP grade water | 805.74 | | | |
| 20% tetrasodium EDTA dihydrate solution (CAS 64-02-8) to adjust pH to 7.4 | 212.75 | 0.2 | 42.55 | 0.04 |
| Lactic acid | | | | |
| | 4564.79 | | 1152.24 | 1.00 |

The pH of the surfactant mixture was 7.4.

### Treatment Mixture B

The following ingredients were combined in the amounts shown. Care was taken to avoid air entrainment and foaming during the mixing operation.

| Ingredient | Amount (grams) | Concentration (gms of ingredient per gm of solution ) delivered by supplier | solids (active ingredient gms) | Fraction of total solids in product |
|---|---|---|---|---|
| Zinc gluconate powder (to deliver 0.5% Zn) 22g Zn | 155 | 0.14 | 155 | 0.14 |
| Sorbitol 70% solution | 52.32 | 0.7 | 36.63 | 0.03 |
| Sodium Laureth Sulfate CAS 68585-34-2 (26% solution in water) | 1465 | 0.26 | 380.90 | 0.34 |
| Sodium Lauryl Sulfoacetate (CAS 1847-58-1) and Disodium Laureth Sulfosuccinate (CAS 39354-45-5) = STEPAN MILD^{©} LSB (25% solution in water) | 1465 | 0.25 | 366.25 | 0.33 |
| Cocodimethylaminopropylbetaine, CAS 70851-07-9, 35% in water | 366.25 | 0.35 | 128.19 | 0.11 |
| Imidazolidinyl urea (preservative) (Germal 115, CAS 39236-46-9) | 14.60 | 1 | 14.60 | 0.01 |
| USP grade water | 805.74 | | | |
| 20% tetrasodium EDTA dihydrate solution (CAS 64-02-8) to adjust pH to 7.4 | 212.75 | 0.2 | 42.55 | 0.04 |
| Lactic acid | | | | |
| | 4536.66 | | 1124.12 | 1.00 |

The pH of the surfactant mixture was 7.4.

Other effective antimicrobial additives instead of imidazolidinyl urea include parabens, methylchloroisothiazolinone, and methylisothiazolinone. Bedsore treatment efficacy was excellent with no antimicrobial additive.

### Reference Example 1

Patient 1, a 72 year old male with Type 2 diabetes, developed a skin crack on his left heel that would not heal. After standard wound care methods the crack wound worsened resulting in treatment by a vascular surgeon wherein, after initial treatment cleared the lesion, an initial skin graft was done which failed after about one month. The lesion was cleaned again and another skin graft was applied which failed resulting in a 3 cm grossly infected left ankle and heel leading to a recommendation for amputation of the ankle and foot. The wound was then treated twice a day with various antibiotics and antibiotic creams and wet to dry packs with no improvement.

Treatment Mixture A was then applied twice per day via a squirt bottle to the sore after cleaning and before the application of a wet to dry pack. Healing commenced shortly after the start of the treatment with Treatment Mixture A, the sore gradually reduced in size, and the patient's sore was totally healed within about 12 weeks.

### Reference Example 2

Patient 2, a 57 year old male, had diabetes mellitus and severe neuropathy of his left foot and leg. His right leg had been amputated due to infection secondary to his diabetes illness. A 4x4 cm blister lesion was present on the plantar surface of his left foot. The lesion had purulent discharge. Amputation of the left foot was recommended.

Topical treatment of the lesion with Treatment Mixture A was begun along with a course of oral antibiotics. Just prior the treatment with Treatment Mixture A, he had purulent discharge and was debrided. One week later after treatment with Treatment Mixture A, the bedsore was healing with no drainage. After five more weeks of treatment, the wound was totally closed and after one additional week, the ulcer was healed and the patient was able to walk with his prosthetic leg as usual.

### Reference Example 3

Patient 3, a 22 year old male, was in poor health, smoked, and had a severe ulcer of over eighteen months duration on his leg. Physicians recommended treatment by a hemipelvectomy. His ulcer was treated erratically with diluted Treatment Mixture A resulting in slow healing. Treatment frequency was then increased to daily, and healing accelerated.

### Reference Example 4

A 70 year old male with poor circulation in the lower limb and feet presented with an abscess on his foot that he had over a year. The abscess had developed an infection that was draining from his 1^{st} and 2^{nd} toe. He was provided with Treatment Mixture A which was diluted by adding 1 ml of Treatment Mixture A into 100 ml of saline solution and rinsing the sores with the diluted mixture. After about 3 - 4 weeks, both toes healed.

### Example 5

A patient such as a 60 year old male with an amputated foot would normally require about 6-8 weeks to heal with conventional wound treatment. The same patient treated with an inventive composition would be expected to heal in much less time.

The present invention is set out in the appended set of claims. While the invention has been described with reference to the preferred embodiments other embodiments and improvements can be realized which are not specifically set forth but which are within the scope of the claimed invention as set fort in the claims appended hereto.

## Claims

1. A treatment mixture for use in the removal of bacterial biofilms on a human or animal body, wherein said treatment mixture can be applied and wherein said biofilm is integral to a bedsore on a human or animal body wherein the treatment mixture compises:
sodium laureth sulfate, 26% solution in water, at an amount of 34 wt% of the solids;
sodium lauryl sulfoacetate and disodium laureth sulfosuccinate, 25% solution in water, at an amount of 33 wt% of the solids;
zinc gluconate powder, at an amount of 14 wt% of the solids;
sorbitol, 70% solution, at an amount of 3 wt% of the solids;
cocodimethylaminopropylbetaine, 35% in water, at an amount of 11 wt% of the solids;
imidazolidinyl urea, at an amount of 1 wt% of the solids;
tetrasodium EDTA dihydrate solution to adjust pH to 7.4, at an amount of 4 wt% of the solids; and
water.

2. The treatment mixture for use according to claim 1, wherein said treatment mixture is selected from a liquid, a gel and a foam;
and/or wherein said treatment mixture comprises nonionic or amphoteric surfactants.

3. The treatment mixture for use according to any preceeding claim in which application of the treatment mixture to a wound on a human or animal body is chosen from the group comprising a spray, soaking on a gauze pad, and application of a gel.

## Patentansprüche

1. Behandlungsgemisch zur Verwendung beim Entfernen von bakteriellen Biofilmen auf einem menschlichen oder tierischen Körper, wobei das genannte Behandlungsgemisch aufgetragen werden kann und wobei der genannte Biofilm integraler Bestandteil eines Dekubitus auf einem menschlichen oder tierischen Körper ist, wobei das Behandlungsgemisch Folgendes umfasst:
Natriumlaurethsulfat, 26%ige Lösung in Wasser, in einer Menge von 34 Gew.-% der Feststoffe;
Natriumlaurylsulfoacetat und Dinatriumlaurethsulfosuccinat, 25%ige Lösung in Wasser, in einer Menge von 33 Gew.-% der Feststoffe;
Zinkgluconatpulver in einer Menge von 14 Gew.-% der Feststoffe;
Sorbitol, 70%ige Lösung, in einer Menge von 3 Gew.-% der Feststoffe;
Cocodimethylaminopropylbetain, 35 % in Wasser, in einer Menge von 11 Gew.-% der Feststoffe;
Imidazolidinylharnstoff in einer Menge von 1 Gew.-% der Feststoffe;
Tetranatrium-EDTA-Dihydratlösung zur Einstellung des pH-Werts auf 7,4 in einer Menge von 4 Gew.-% der Feststoffe; und
Wasser.

2. Behandlungsgemisch zur Verwendung nach Anspruch 1, wobei das genannte Behandlungsgemisch aus einer Flüssigkeit, einem Gel und einem Schaum ausgewählt ist;
und/oder wobei das genannte Behandlungsgemisch nichtionische oder amphotere Tenside umfasst.

3. Behandlungsgemisch zur Verwendung nach einem vorherigen Anspruch, wobei die Anwendung des Behandlungsgemischs auf eine Wunde am menschlichen oder tierischen Körper aus der Gruppe ausgewählt ist, die Aufsprühen, Einweichen auf einem Mulltupfer und Auftragen eines Gels umfasst.

## Revendications

1. Mélange de traitement destiné à servir dans l'élimination de biofilms bactériens sur un corps humain ou animal, ledit mélange de traitement pouvant être appliqué et dans lequel ledit biofilm fait partie intégrante d'une escarre sur un corps humain ou animal, le mélange de traitement comprenant :
une solution de sulfate de laureth sodique à 26 % dans l'eau, à une quantité de 34 % en poids des solides ;
une solution de sodium lauryl sulfoacétate et de dissodium laureth sulfosuccinate à 25 % dans l'eau, à une quantité de 33 % en poids des solides ;
une poudre de gluconate de zinc, à une quantité de 14 % en poids des solides ;
une solution de sorbitol à 70 %, à une quantité de 3 % en poids des solides ;
du cocodiméthylaminopropylbétaïne, à 35 % d'eau, à une quantité de 1,1 % en poids des solides ;
de l'imidazolidinyl urée, à une quantité de 1 % en poids des solides ;
une solution de tétrasodium EDTA dihydraté pour ajuster le pH à 7,4, à une quantité de 4 % en poids des solides ; et
de l'eau.

2. Mélange de traitement destiné à servir selon la revendication 1, ledit mélange de traitement étant sélectionné parmi un liquide, un gel et une mousse ;
et/ou ledit mélange de traitement comprend des tensioactifs non ioniques ou amphotériques.

3. Mélange de traitement destiné à servir selon toute revendication précédente dans lequel l'application du mélange de traitement sur une plaie sur un corps humain ou animal est sélectionnée dans le groupe comprenant un spray, un trempage sur une compresse de gaze et l'application d'un gel.
